# EUROPEAN PATENT APPLICATION

(11) **EP 3 009 147 A1**
(43) Date of publication of application: **20.04.2016**
(21) Application number: 14306644.7
(22) Date of filing: 16.10.2014
(51) Int. Cl.: A61K 39/395, C07K 16/28, C12Q 1/68, C12N 15/113, G01N 33/574, A61P 35/00

(54) **Method for treating resistant glioblastoma**

(71) Applicant: INSERM (Institut National de la Santé et de la Recherche Médicale), 75013 Paris (FR); Université Paul Sabatier (Toulouse III), 31400 Toulouse (FR)
(72) Inventor: The designation of the inventor has not yet been filed
(74) Representative: Hirsch, Denise Marie

(57) **Abstract**

The present invention relates to a compound which is an antagonist of β8 integrin or an inhibitor of the expression of β8 integrin for use in the treatment of resistant glioblastoma.

## Description

### FIELD OF THE INVENTION:

The present invention relates to a compound which is an antagonist of β8 integrin or an inhibitor of the expression of β8 integrin for use in the treatment of resistant glioblastoma.

### BACKGROUND OF THE INVENTION:

Glioblastomas (GBM) are the most common and malignant primary brain tumors in adults, and account for more than 50% of malignant gliomas. Since 2005, the standard therapy for this tumor includes surgery followed by radiotherapy to 60 Gy in combination with temozolomide (TMZ), allowing an improved survival (Stupp R. et al., 2005). Nevertheless, the prognosis of the patients with a GBM remains very bad, with a median survival of 14.6 months, and a 2-year survival rate of 27.2%, because of a local recurrence mainly due to resistance of the GBM cells to radiotherapy.

Glioblastoma, some malignant, invasive and radio/chemoresistant brain tumors, are characterized by prompt relapse. These aggressive properties at least involve, among these heterogeneous tumors, a subpopulation of highly tumorigenic and radioresistant glioblastoma stem-like cells (GSC). Current research focuses therefore on a specific targeting of this population but there is a need in new therapeutic compounds to treat resistant glioblastoma.

### SUMMARY OF THE INVENTION:

The inventors showed that β8 integrin is overexpressed in glioblastoma stem cells (GSC) and appears to be associated with specific features and functionality to the GSC, including their radiation resistance.

Thus, the invention relates to a compound which is an antagonist of β8 integrin or an inhibitor of the expression of β8 integrin for use in the treatment of resistant glioblastoma.

### DETAILED DESCRIPTION OF THE INVENTION:

### Therapeutic methods

A first object of the invention relates to a compound which is an antagonist of β8 integrin or an inhibitor of the expression of β8 integrin for use in the treatment of resistant glioblastoma.

In one embodiment, the resistant glioblastoma is a radioresistant glioblastoma or a chemoresistant glioblastoma.

In another embodiment, said compound according to the invention is a β8 integrin antagonist.

In another embodiment, the glioblastoma is resistant to treatment using chemotherapeutic compound like Tipifarnib, Temozolomide, Cilengitide (see Stupp R et al., 2014), Bevacizumab (Thomas AA et al., 2014) and EGFR inhibitors like Erlotinib, Gefitinib or Cetuximab.

In another embodiment, the invention relates to a compound which is an antagonist of β8 integrin or an inhibitor of the expression of β8 integrin for use in the treatment of glioblastoma which are characterized by the enrichment of stem cells (GSC) in glioblastoma.

According to the invention, the compound may also be an inhibitor of a molecular complex containing β8 integrin or an inhibitor of β8 integrin signaling pathway.

As used herein, the term "molecular complex containing β8 integrin" denotes a macromolecular structure formed by the functional β8 integrin present at the cellular membrane and any adaptors, effectors proteins and/or ligands at the extracellular and/or intracellular interface and which is required to mediate any effects or functions of β8 integrin.

As used herein the term "Glioblastoma Stem Cells (GSC)" denotes a subpopulation of highly tumorigenic and radioresistant stem cells present in the brain tumor. These cells can express CD 133 gene and/or a panel of other genes characteristic of neural stem cells (Nestin, A2B5, Olig2, Sox2, etc...) and possess the self-renewal potential (see Cheng L et al. 2010).

Thus as used herein, the term "enrichment of stem cells (GSC) in glioblastoma" denotes an over-presence of GSC in glioblastoma.

As used herein, the term "β8 integrin" has it general meaning in the art and refers to a member of the integrin's family which are receptors that mediate attachment between a cell and the tissues surrounding it, which may be other cells or the extracellular matrix (ECM). They also play a role in cell signaling and thereby regulate cellular shape, motility, invasion, angiogenesis, survival and the cell cycle.

In one embodiment, the compound according to the invention may binds to β8 integrin or β8 integrin complex or inhibits β8 integrin expression, and blocks the related physiological effects. To identify a compound able to block this molecule, cell adhesion assays on selective β8 integrin substrates (fibronectin, vitronectin for example) will be performed as well as the measurement of extracellular TGF-β activation, a downstream selective event to β8 integrin involvement (Cambier et al Am J Pathol 2005). Intracellular downstream events to β8 integrin activation will also be analyzed (interaction with RhoGDI1, Reyes et al Mol Biol Cell 2013, or activation of the MAPK pathway) in response to its inhibition. Finally, β8 integrin mRNA expression level and membrane expression of this protein will be assessed by qPCR and flow cytometry respectively to monitor the effect of inhibitors of β8 integrin expression. Other validation systems could also be used to assess the inhibition/interaction of the aforementioned β8 integrin inhibitor with its target, notably through cell free methods. For example, ELISA techniques using purified β8 integrin could also be used.

In a particular embodiment, the compound according to the invention may be used to improve the sensitivity of glioblastoma to different chemotherapeutic agents or to enhance the sensitivity of glioblastoma to radiotherapy, or to improve glioblastoma sensitivity to associated radio/chemotherapy.

Thus, the invention also relates to a compound which is an antagonist of β8 integrin or an inhibitor of the expression of β8 integrin for use to improve the sensitivity of glioblastoma to radiotherapy.

In another particular embodiment, the compound according to the invention is administrated in combination with radiotherapy.

Thus, the invention also relates to i) compound according to the invention, and ii) a radiotherapy, as a combined preparation for simultaneous, separate or sequential for use in the treatment of resistant glioblastoma.

In other word, the invention also relates to i) compound according to the invention, and ii) a radiotherapeutic agent, as a combined preparation for simultaneous, separate or sequential for use in the treatment of resistant glioblastoma.

In another embodiment, the invention also relates to i) compound according to the invention, and ii) a radiotherapeutic agent, as a combined preparation for simultaneous, separate or sequential to improve the sensitivity of resistant glioblastoma to radiotherapy.

In another particular embodiment, the compound according to the invention is administrated in combination with a chemotherapeutic agent.

Thus, the invention also relates to i) compound according to the invention, and ii) a chemotherapeutic agent, as a combined preparation for simultaneous, separate or sequential for use in the treatment of resistant glioblastoma.

In another embodiment, the invention also relates to i) compound according to the invention, and ii) chemotherapeutic agent, as a combined preparation for simultaneous, separate or sequential to improve the sensitivity of resistant glioblastoma to radiotherapy.

In another particular embodiment, the compound according to the invention is administrated in combination with radiotherapy and temozolomide (TMZ).

Thus, the invention also relates to i) compound according to the invention, and ii) a radiotherapy, and iii) TMZ as a combined preparation for simultaneous, separate or sequential use in the treatment of resistant glioblastoma.

In other word, the invention also relates to i) compound according to the invention, ii) a radiotherapeutic agent, and iii) TMZ as a combined preparation for simultaneous, separate or sequential for use in the treatment of resistant glioblastoma.

In another embodiment, the invention also relates to i) compound according to the invention, ii) a radiotherapeutic agent, and iii) TMZ as a combined preparation for simultaneous, separate or sequential to improve the sensitivity of resistant glioblastoma to radiotherapy.

As used herein, "radiotherapy" may consist of gamma-radiation, X-ray radiation, electrons or photons, external radiotherapy or curitherapy.

As used herein, the term "radiotherapeutic agent", is intended to refer to any radiotherapeutic agent known to one of skill in the art to be effective to treat or ameliorate cancer, without limitation. For instance, the radiotherapeutic agent can be an agent such as those administered in brachytherapy or radionuclide therapy. Such methods can optionally further comprise the administration of one or more additional cancer therapies, such as, but not limited to, chemotherapies, and/or another radiotherapy.

In another particular embodiment, the invention also relates to a compound which is an antagonist of β8 integrin or an inhibitor of the expression of β8 integrin for use in the treatment of glioblastoma.

In one embodiment, said β8 integrin, antagonist may be a low molecular weight antagonist, e. g. a small organic molecule (natural or not).

The term "small organic molecule" refers to a molecule (natural or not) of a size comparable to those organic molecules generally used in pharmaceuticals. The term excludes biological macromolecules (e. g., proteins, nucleic acids, etc.). Preferred small organic molecules range in size up to about 10000 Da, more preferably up to 5000 Da, more preferably up to 2000 Da and most preferably up to about 1000 Da.

Antagonists of β8 integrin are well known in the state of the art (see for example Minagawa S et al., 2014 or Sheldrake HM et al., 2014).

In another embodiment, β8 integrin, antagonist of the invention may be an anti-β8 integrin antibody which neutralizes β8 integrin or an anti-β8 integrin fragment thereof which neutralizes β8 integrin, or an anti- β8 integrin nanobody.

Antibodies directed against β8 integrin can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred. Monoclonal antibodies against β8 integrin can be prepared and isolated using any technique that provides for the production of antibody molecules by continuous cell lines in culture. Techniques for production and isolation include but are not limited to the hybridoma technique originally described by Kohler and Milstein (1975); the human B-cell hybridoma technique (Cote et al., 1983); and the EBV-hybridoma technique (Cole et al. 1985). Alternatively, techniques described for the production of single chain antibodies (see e.g., U.S. Pat. No. 4,946,778) can be adapted to produce anti-β8 integrin single chain antibodies. β8 integrin antagonists useful in practicing the present invention also include antibody fragments including but not limited to F(ab')2 fragments, which can be generated by pepsin digestion of an intact antibody molecule, and Fab fragments, which can be generated by reducing the disulfide bridges of the F(ab')2 fragments. Alternatively, Fab and/or scFv expression libraries can be constructed to allow rapid identification of fragments having the desired specificity to β8 integrin.

Humanized anti-β8 integrin antibodies and antibody fragments therefrom can also be prepared according to known techniques. "Humanized antibodies" are forms of non-human (e.g., rodent) chimeric antibodies that contain minimal sequence derived from non-human immunoglobulin. For the most part, humanized antibodies are human immunoglobulins (recipient antibody) in which residues from a hypervariable region (CDRs) of the recipient are replaced by residues from a hypervariable region of a non-human species (donor antibody) such as mouse, rat, rabbit or nonhuman primate having the desired specificity, affinity and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. Methods for making humanized antibodies are described, for example, by Winter (U.S. Pat. No. 5,225,539) and Boss (Celltech, U.S. Pat. No. 4,816,397).

Then, for this invention, neutralizing antibodies of β8 integrin are selected.

In a particular embodiment, the anti-β8 integrin antibody according to the invention is an antibody selected in the patent application WO2013026004.

In a particular embodiment, the anti-β8 integrin antibody according to the invention is an antibody selected in the patent application WO 2011103490

In a particular embodiment, the anti-β8 integrin antibody according to the invention is an antibody selected in the patent applications US5635601 or CA2790488.

In a particular embodiment, the anti-β8 integrin antibody according to the invention is an anti-αVβ8 antibody selected in the patent application WO2011020529.

In a particular embodiment, the anti-β8 integrin antibody according to the invention is the antibody MAB4775 obtained by the society R&D or the antibodies ab172007 and ab80673 obtained by the society Abcam or the antibody sc-25714 obtained by the society Santa Cruz.

In still another embodiment, β8 integrin antagonists may be selected from aptamers. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. and Gold L., 1990. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consists of a conformationally constrained antibody variable region displayed by a platform protein, such as E. coli Thioredoxin A that are selected from combinatorial libraries by two hybrid methods (Colas et al., 1996).

Then, for this invention, neutralizing aptamers of β8 integrin are selected.

In another embodiment, the compound according to the invention may be selected from peptides, peptides mimetic or RGD peptide mimetics (see Sheldrake HM et al., 2014).

In a preferred embodiment, the compound according to the invention is an inhibitor of the β8 integrin gene expression.

Small inhibitory RNAs (siRNAs) can also function as inhibitors of β8 integrin gene expression for use in the present invention. β8 integrin gene expression can be reduced by contacting a subject or cell with a small double stranded RNA (dsRNA), or a vector or construct causing the production of a small double stranded RNA, such that β8 integrin gene expression is specifically inhibited (i.e. RNA interference or RNAi). Methods for selecting an appropriate dsRNA or dsRNA-encoding vector are well known in the art for genes whose sequence is known (e.g. see for example Tuschl, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836).

Ribozymes can also function as inhibitors of β8 integrin gene expression for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of β8 integrin mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, e.g., ribonuclease protection assays.

Both antisense oligonucleotides and ribozymes useful as inhibitors of β8 integrin gene expression can be prepared by known methods. These include techniques for chemical synthesis such as, e.g., by solid phase phosphoramadite chemical synthesis. Alternatively, anti-sense RNA molecules can be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone.

Antisense oligonucleotides siRNAs and ribozymes of the invention may be delivered in vivo alone or in association with a vector. In its broadest sense, a "vector" is any vehicle capable of facilitating the transfer of the antisense oligonucleotide siRNA or ribozyme nucleic acid to the cells and preferably cells expressing β8 integrin. Preferably, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the antisense oligonucleotide siRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, 1990 and in Murry, 1991).

Preferred viruses for certain applications are the adeno-viruses and adeno-associated viruses, which are double-stranded DNA viruses that have already been approved for human use in gene therapy. The adeno-associated virus can be engineered to be replication deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. See e.g. Sambrook et al., 1989. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, eye, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

In a preferred embodiment, the antisense oligonucleotide, siRNA, shRNA or ribozyme nucleic acid sequence is under the control of a heterologous regulatory region, e.g., a heterologous promoter. The promoter may be specific for Muller glial cells, microglia cells, endothelial cells, pericyte cells and astrocytes. For example, a specific expression in Muller glial cells may be obtained through the promoter of the glutamine synthetase gene is suitable. The promoter can also be, e.g., a viral promoter, such as CMV promoter or any synthetic promoters.

Another object of the invention relates to a method for treating resistant glioblastoma comprising administering to a subject in need thereof a therapeutically effective amount of a compound which is an antagonist of β8 integrin or an inhibitor of the expression of β8 integrin as described above.

Compounds of the invention may be administered in the form of a pharmaceutical composition, as defined below.

By a "therapeutically effective amount" is meant a sufficient amount of compound to treat resistant glioblastoma disorder.

It will be understood that the total daily usage of the compounds and compositions of the present invention will be decided by the attending physician within the scope of sound medical judgment and/or during phase 1 clinical trial. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

Compounds according to the invention may be used for the preparation of a pharmaceutical composition for the treatment of resistant glioblastoma.

Hence, the present invention also provides a pharmaceutical composition comprising an effective dose of an antagonist of β8 integrin or an inhibitor of the β8 integrin expression, particularly a β8 integrin antagonist, according to the invention.

Any therapeutic agent of the invention may be combined with pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The form of the pharmaceutical compositions, the route of administration, the dosage and the regimen naturally depend upon the condition to be treated, the severity of the illness, the age, weight, and sex of the patient, etc.

The pharmaceutical compositions of the invention can be formulated for a topical, oral, intranasal, parenteral, intraocular, intravenous, intramuscular, intratumoral or subcutaneous administration and the like.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.

In addition, other pharmaceutically acceptable forms include, e.g. tablets or other solids for oral administration; time release capsules; and any other form currently can be used.

Compositions of the present invention may comprise a further therapeutic active agent. The present invention also relates to a kit comprising an antagonist or an inhibitor according to the invention and a further therapeutic active agent.

In one embodiment said therapeutic active agent is an anticancer agent. For example, said anticancer agents include but are not limited to fludarabine, gemcitabine, capecitabine, methotrexate, taxol, taxotere, mercaptopurine, thioguanine, hydroxyurea, cytarabine, cyclophosphamide, ifosfamide, nitrosoureas, platinum complexes such as cisplatin, carboplatin and oxaliplatin, mitomycin, dacarbazine, procarbizine, etoposide, teniposide, campathecins, bleomycin, doxorubicin, idarubicin, daunorubicin, dactinomycin, plicamycin, mitoxantrone, L-asparaginase, doxorubicin, epimbicm, 5-fluorouracil, taxanes such as docetaxel and paclitaxel, leucovorin, levamisole, irinotecan, estramustine, etoposide, nitrogen mustards, BCNU, nitrosoureas such as carmustme and lomustine, vinca alkaloids such as vinblastine, vincristine and vinorelbine, imatimb mesylate, hexamethyhnelamine, topotecan, kinase inhibitors, phosphatase inhibitors, ATPase inhibitors, tyrphostins, protease inhibitors, inhibitors herbimycm A, genistein, erbstatin, temolozomide and lavendustin A. In one embodiment, additional anticancer agents may be selected from, but are not limited to, one or a combination of the following class of agents: alkylating agents, plant alkaloids, DNA topoisomerase inhibitors, anti-folates, pyrimidine analogs, purine analogs, DNA antimetabolites, taxanes, podophyllotoxin, hormonal therapies, retinoids, photosensitizers or photodynamic therapies, angiogenesis inhibitors, antimitotic agents, isoprenylation inhibitors, cell cycle inhibitors, actinomycins, bleomycins, anthracyclines, MDR inhibitors and Ca2+ ATPase inhibitors.

Additional anticancer agents may be selected from, but are not limited to, cytokines, chemokines, growth factors, growth inhibitory factors, hormones, soluble receptors, decoy receptors, monoclonal or polyclonal antibodies, mono-specific, bi-specific or multi-specific antibodies, monobodies, polybodies.

Additional anticancer agent may be selected from, but are not limited to, growth or hematopoietic factors such as erythropoietin and thrombopoietin, and growth factor mimetics thereof.

In the present methods for treating cancer the further therapeutic active agent can be an antiemetic agent. Suitable antiemetic agents include, but are not limited to, metoclopromide, domperidone, prochlorperazine, promethazine, chlorpromazine, trimethobenzamide, ondansetron, granisetron, hydroxyzine, acethylleucine monoemanolamine, alizapride, azasetron, benzquinamide, bietanautine, bromopride, buclizine, clebopride, cyclizine, dunenhydrinate, diphenidol, dolasetron, meclizme, methallatal, metopimazine, nabilone, oxypemdyl, pipamazine, scopolamine, sulpiride, tetrahydrocannabinols, thiefhylperazine, thioproperazine and tropisetron. In a preferred embodiment, the antiemetic agent is granisetron or ondansetron.

In another embodiment, the further therapeutic active agent can be an hematopoietic colony stimulating factor. Suitable hematopoietic colony stimulating factors include, but are not limited to, filgrastim, sargramostim, molgramostim and epoietin alpha.

In still another embodiment, the other therapeutic active agent can be an opioid or non-opioid analgesic agent. Suitable opioid analgesic agents include, but are not limited to, morphine, heroin, hydromorphone, hydrocodone, oxymorphone, oxycodone, metopon, apomorphine, nomioiphine, etoipbine, buprenorphine, mepeddine, lopermide, anileddine, ethoheptazine, piminidine, betaprodine, diphenoxylate, fentanil, sufentanil, alfentanil, remifentanil, levorphanol, dextromethorphan, phenazodne, pemazocine, cyclazocine, methadone, isomethadone and propoxyphene. Suitable non-opioid analgesic agents include, but are not limited to, aspirin, celecoxib, rofecoxib, diclofinac, diflusinal, etodolac, fenoprofen, flurbiprofen, ibuprofen, ketoprofen, indomethacin, ketorolac, meclofenamate, mefanamic acid, nabumetone, naproxen, piroxicam and sulindac.

### Diagnostic methods

In another aspect, the invention relates to an ex vivo method for diagnosing resistant glioblastoma, comprising the step of determining the level expression of the marker β8 integrin in a tumor sample obtained from a patient.

Typically, the tumor sample according to the invention may be a cancer biopsy or a cancer biopsy obtained during surgery.

The term "detecting" as used above includes qualitative and/or quantitative detection (measuring levels) with or without reference to a control. Typically β8 integrin expression may be measured for example by enzyme-labeled and mediated immunoassays (such as ELISA) or by flow cytometry performed on the sample. Moreover, FACS sorting could additionally be performed on β8 integrin positive tumoral cells.

Particularly, the invention relates to a method for diagnosis of resistant glioblastoma in a patient comprising a step a) consisting of measuring β8 integrin expression level in a sample obtained from said patient. Preferably, the method of the invention further comprises a step of comparing the β8 integrin expression level obtained in step a) to a threshold value.

The "control" may be a healthy subject, i.e. a subject who does not suffer from any resistant glioblastoma. The control may also be a subject suffering from resistant glioblastoma. Preferably, said control is a healthy subject.

Detection of β8 integrin expression in the sample may be performed by measuring the level of β8 integrin protein. In the present application, the "level of β8 integrin protein" means the quantity or concentration of said β8 integrin protein.

Such methods comprise contacting a sample with a binding partner capable of selectively interacting with β8 integrin protein present in the sample. The binding partner is generally an antibody that may be polyclonal or monoclonal, preferably monoclonal.

The presence of the protein can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, immunohistochemistry, etc. The reactions generally include revealing labels such as fluorescent, chemiluminescent, radioactive, enzymatic labels or dye molecules, or other methods for detecting the formation of a complex between the antigen and the antibody or antibodies reacted therewith.

The aforementioned assays generally involve separation of unbound protein in a liquid phase from a solid phase support to which antigen-antibody complexes are bound. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like.

More particularly, an ELISA method can be used, wherein the wells of a microtiter plate are coated with a set of antibodies against the proteins to be tested. A sample containing or suspected of containing the marker protein is then added to the coated wells. After a period of incubation sufficient to allow the formation of antibody-antigen complexes, the plate(s) can be washed to remove unbound moieties and a detectably labeled secondary binding molecule is added. The secondary binding molecule is allowed to react with any captured sample marker protein, the plate is washed and the presence of the secondary binding molecule is detected using methods well known in the art.

Various immunoenzymatic staining methods are known in the art for detecting a protein of interest. For example, immunoenzymatic interactions can be visualized using different enzymes such as peroxidase, alkaline phosphatase, or different chromogens such as DAB, AEC, or Fast Red; or fluorescent labels such as FITC, Cy3, Cy5, Cy7, Alexafluors, APC, etc. Counterstains may include H&E, DAPI, Hoechst, so long as such stains are compatible with other detection reagents and the visualization strategy used. As known in the art, amplification reagents may be used to intensify staining signal. For example, tyramide reagents may be used. The staining methods of the present invention may be accomplished using any suitable method or system as would be apparent to one of skill in the art, including automated, semi-automated or manual systems.

The method of the invention may comprise a further step consisting of comparing β8 integrin expression with a control reference.

The invention thus relates to a method for diagnosis resistant glioblastoma in a patient comprising determining the expression level of β8 integrin in a sample obtained from said patient and comparing said expression level to a threshold value. As used herein, "expression level of β8 integrin " refers to an amount of a translation product, for instance the protein β8 integrin. Typically, a level of mRNA expression can be expressed in units such as transcripts per cell or nanograms per microgram of tissue. A level of a polypeptide can be expressed as nanograms per microgram of tissue or nanograms per milliliter of a culture medium, for example. Alternatively, relative units can be employed to describe an expression level.

Typically, a "threshold value", "threshold level" or "cut-off value" can be determined experimentally, empirically, or theoretically. A threshold value can also be arbitrarily selected based upon the existing experimental and/or clinical conditions, as would be recognized by a person of ordinary skilled in the art. Preferably, the person skilled in the art may compare the expression levels of β8 integrin obtained according to the method of the invention with a defined threshold value.

Preferably, said threshold value is the mean expression level of β8 integrin of a population of healthy individuals. As used herein, the term "healthy individual" denotes a human which is known to be healthy, i.e. which does not suffer from resistant glioblastoma, has never been subjected to such resistant glioblastoma, and does not need any medical care.

Typically, the skilled person in the art may determine the expression level of β8 integrin in a biological sample of 100 individuals known to be healthy. The mean value of the obtained expression levels is then determined, according to well known statistical analysis, so as to obtain the mean expression level of β8 integrin. Said value is then considered as being normal and thus constitute a threshold value. By comparing the expression levels of β8 integrin to this threshold value, the physician is then able to diagnose resistant glioblastoma. Indeed, by comparing the expression level of β8 integrin obtained in a biological sample, of a given subject to a threshold value, one can easily determine whether said subject suffers from resistant glioblastoma or not.

Accordingly, the physician would be able to adapt and optimize appropriate medical care of a subject in a critical and life-threatening condition suffering from resistant glioblastoma. The determination of said prognosis is highly appropriate for follow-up care and clinical decision making.

Therefore, the invention is drawn to a method for diagnosis resistant glioblastoma in a patient comprising the following steps:
a) determining the level of expression of β8 integrin in a sample obtained from said patient;
b) determining the mean expression level of β8 integrin in a biological sample of a population of healthy individuals, preferably 100 healthy individuals; and
c) a step of comparing the expression level of β8 integrin obtained of a) to the mean expression level of β8 integrin obtained in b).

The present invention also relates to kits for the diagnosis of resistant glioblastoma, comprising means for detecting β8 integrin expression.

According to the invention, the kits of the invention may comprise an anti- β8 integrin protein antibody; and another molecule coupled with a signalling system which binds to said β8 integrin protein antibody.

Typically, the antibodies or combination of antibodies are in the form of solutions ready for use. In one embodiment, the kit comprises containers with the solutions ready for use. Any other forms are encompassed by the present invention and the man skilled in the art can routinely adapt the form to the use in immunohistochemistry.

In another embodiment, the expression level of the gene of β8 integrin can be done. Measuring the expression level of a gene can be performed by a variety of techniques well known in the art.

Typically, the expression level of a gene may be determined by determining the quantity of mRNA. Methods for determining the quantity of mRNA are well known in the art. For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (e. g., Northern blot analysis, in situ hybridization) and/or amplification (e.g., RT-PCR).

Other methods of Amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization.

Typically, the nucleic acid probes include one or more labels, for example to permit detection of a target nucleic acid molecule using the disclosed probes. In various applications, such as in situ hybridization procedures, a nucleic acid probe includes a label (e.g., a detectable label). A "detectable label" is a molecule or material that can be used to produce a detectable signal that indicates the presence or concentration of the probe (particularly the bound or hybridized probe) in a sample. Thus, a labeled nucleic acid molecule provides an indicator of the presence or concentration of a target nucleic acid sequence (e.g., genomic target nucleic acid sequence) (to which the labeled uniquely specific nucleic acid molecule is bound or hybridized) in a sample. A label associated with one or more nucleic acid molecules (such as a probe generated by the disclosed methods) can be detected either directly or indirectly. A label can be detected by any known or yet to be discovered mechanism including absorption, emission and/ or scattering of a photon (including radio frequency, microwave frequency, infrared frequency, visible frequency and ultra-violet frequency photons). Detectable labels include colored, fluorescent, phosphorescent and luminescent molecules and materials, catalysts (such as enzymes) that convert one substance into another substance to provide a detectable difference (such as by converting a colorless substance into a colored substance or vice versa, or by producing a precipitate or increasing sample turbidity), haptens that can be detected by antibody binding interactions, and paramagnetic and magnetic molecules or materials.

Particular examples of detectable labels include fluorescent molecules (or fluorochromes). Numerous fluorochromes are known to those of skill in the art, and can be selected, for example from Life Technologies (formerly Invitrogen), e.g., see, The Handbook-A Guide to Fluorescent Probes and Labeling Technologies). Examples of particular fluorophores that can be attached (for example, chemically conjugated) to a nucleic acid molecule (such as a uniquely specific binding region) are provided in U.S. Pat. No. 5,866, 366 to Nazarenko et al., such as 4-acetamido-4'-isothiocyanatostilbene-2,2' disulfonic acid, acridine and derivatives such as acridine and acridine isothiocyanate, 5-(2'-aminoethyl) amino naphthalene-1-sulfonic acid (EDANS), 4-amino -N- [3 vinylsulfonyl)phenyl]naphthalimide-3,5 disulfonate (Lucifer Yellow VS), N-(4-anilino-1-naphthyl)maleimide, antl1ranilamide, Brilliant Yellow, coumarin and derivatives such as coumarin, 7-amino-4-methylcoumarin (AMC, Coumarin 120), 7-amino-4-trifluoromethylcouluarin (Coumarin 151); cyanosine; 4',6-diaminidino-2-phenylindole (DAPI); 5',5"dibromopyrogallol-sulfonephthalein (Bromopyrogallol Red); 7 -diethylamino -3 - (4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulforlic acid; 5-[dimethylamino] naphthalene-1-sulfonyl chloride (DNS, dansyl chloride); 4-(4'-dimethylaminophenylazo)benzoic acid (DABCYL); 4-dimethylaminophenylazophenyl-4'-isothiocyanate (DABITC); eosin and derivatives such as eosin and eosin isothiocyanate; erythrosin and derivatives such as erythrosin B and erythrosin isothiocyanate; ethidium; fluorescein and derivatives such as 5-carboxyfluorescein (FAM), 5-(4,6dicl1lorotriazin-2-yDarninofluorescein (DTAF), 2'7'dimethoxy-4'5'-dichloro-6-carboxyfluorescein (JOE), fluorescein, fluorescein isothiocyanate (FITC), and QFITC Q(RITC); 2',7'-difluorofluorescein (OREGON GREEN®); fluorescamine; IR144; IR1446; Malachite Green isothiocyanate; 4-methylumbelliferone; ortho cresolphthalein; nitrotyrosine; pararosaniline; Phenol Red; B-phycoerythrin; o-phthaldialdehyde; pyrene and derivatives such as pyrene, pyrene butyrate and succinimidyl 1-pyrene butyrate; Reactive Red 4 (Cibacron Brilliant Red 3B-A); rhodamine and derivatives such as 6-carboxy-X-rhodamine (ROX), 6-carboxyrhodamine (R6G), lissamine rhodamine B sulfonyl chloride, rhodamine (Rhod), rhodamine B, rhodamine 123, rhodamine X isothiocyanate, rhodamine green, sulforhodamine B, sulforhodamine 101 and sulfonyl chloride derivative of sulforhodamine 101 (Texas Red); N,N,N',N'-tetramethyl-6-carboxyrhodamine (TAMRA); tetramethyl rhodamine; tetramethyl rhodamine isothiocyanate (TRITC); riboflavin; rosolic acid and terbium chelate derivatives. Other suitable fluorophores include thiol-reactive europium chelates which emit at approximately 617 mn (Heyduk and Heyduk, Analyt. Biochem. 248:216-27, 1997; J. Biol. Chem. 274:3315-22, 1999), as well as GFP, LissamineTM, diethylaminocoumarin, fluorescein chlorotriazinyl, naphthofluorescein, 4,7-dichlororhodamine and xanthene (as described in U.S. Pat. No. 5,800,996 to Lee et al.) and derivatives thereof. Other fluorophores known to those skilled in the art can also be used, for example those available from Life Technologies (Invitrogen; Molecular Probes (Eugene, Oreg.)) and including the ALEXA FLUOR® series of dyes (for example, as described in U.S. Pat. Nos. 5,696,157, 6, 130, 101 and 6,716,979), the BODIPY series of dyes (dipyrrometheneboron difluoride dyes, for example as described in U.S. Pat. Nos. 4,774,339, 5,187,288, 5,248,782, 5,274,113, 5,338,854, 5,451,663 and 5,433,896), Cascade Blue (an amine reactive derivative of the sulfonated pyrene described in U.S. Pat. No. 5,132,432) and Marina Blue (U.S. Pat. No. 5,830,912).

In addition to the fluorochromes described above, a fluorescent label can be a fluorescent nanoparticle, such as a semiconductor nanocrystal, e.g., a QUANTUM DOTTM (obtained, for example, from Life Technologies (QuantumDot Corp, Invitrogen Nanocrystal Technologies, Eugene, Oreg.); see also, U.S. Pat. Nos. 6,815,064; 6,682,596; and 6,649, 138). Semiconductor nanocrystals are microscopic particles having size-dependent optical and/or electrical properties. When semiconductor nanocrystals are illuminated with a primary energy source, a secondary emission of energy occurs of a frequency that corresponds to the handgap of the semiconductor material used in the semiconductor nanocrystal. This emission can he detected as colored light of a specific wavelength or fluorescence. Semiconductor nanocrystals with different spectral characteristics are described in e.g., U.S. Pat. No. 6,602,671. Semiconductor nanocrystals that can he coupled to a variety of biological molecules (including dNTPs and/or nucleic acids) or substrates by techniques described in, for example, Bruchez et al., Science 281 :20132016, 1998; Chan et al., Science 281:2016-2018, 1998; and U.S. Pat. No. 6,274,323. Formation of semiconductor nanocrystals of various compositions are disclosed in, e.g., U.S. Pat. Nos. 6,927, 069; 6,914,256; 6,855,202; 6,709,929; 6,689,338; 6,500,622; 6,306,736; 6,225,198; 6,207,392; 6,114,038; 6,048,616; 5,990,479; 5,690,807; 5,571,018; 5,505,928; 5,262,357 and in U.S. Patent Publication No. 2003/0165951 as well as PCT Publication No. 99/26299 (published May 27, 1999). Separate populations of semiconductor nanocrystals can he produced that are identifiable based on their different spectral characteristics. For example, semiconductor nanocrystals can he produced that emit light of different colors hased on their composition, size or size and composition. For example, quantum dots that emit light at different wavelengths based on size (565 mn, 655 mn, 705 mn, or 800 mn emission wavelengths), which are suitable as fluorescent labels in the probes disclosed herein are available from Life Technologies (Carlshad, Calif.).

Additional labels include, for example, radioisotopes (such as 3 H), metal chelates such as DOTA and DPTA chelates of radioactive or paramagnetic metal ions like Gd3+, and liposomes.

Detectable labels that can he used with nucleic acid molecules also include enzymes, for example horseradish peroxidase, alkaline phosphatase, acid phosphatase, glucose oxidase, beta-galactosidase, beta-glucuronidase, or beta-lactamase.

Alternatively, an enzyme can be used in a metallographic detection scheme. For example, silver in situ hybridization (SISH) procedures involve metallographic detection schemes for identification and localization of a hybridized genomic target nucleic acid sequence. Metallographic detection methods include using an enzyme, such as alkaline phosphatase, in combination with a water-soluble metal ion and a redox-inactive substrate of the enzyme. The substrate is converted to a redox-active agent by the enzyme, and the redoxactive agent reduces the metal ion, causing it to form a detectable precipitate. (See, for example, U.S. Patent Application Publication No. 2005/0100976, PCT Publication No. 2005/ 003777 and U.S. Patent Application Publication No. 2004/ 0265922). Metallographic detection methods also include using an oxido-reductase enzyme (such as horseradish peroxidase) along with a water soluble metal ion, an oxidizing agent and a reducing agent, again to form a detectable precipitate. (See, for example, U.S. Pat. No. 6,670,113).

Probes made using the disclosed methods can be used for nucleic acid detection, such as ISH procedures (for example, fluorescence in situ hybridization (FISH), chromogenic in situ hybridization (CISH) and silver in situ hybridization (SISH)) or comparative genomic hybridization (CGH).

In situ hybridization (ISH) involves contacting a sample containing target nucleic acid sequence (e.g., genomic target nucleic acid sequence) in the context of a metaphase or interphase chromosome preparation (such as a cell or tissue sample mounted on a slide) with a labeled probe specifically hybridizable or specific for the target nucleic acid sequence (e.g., genomic target nucleic acid sequence). The slides are optionally pretreated, e.g., to remove paraffin or other materials that can interfere with uniform hybridization. The sample and the probe are both treated, for example by heating to denature the double stranded nucleic acids. The probe (formulated in a suitable hybridization buffer) and the sample are combined, under conditions and for sufficient time to permit hybridization to occur (typically to reach equilibrium). The chromosome preparation is washed to remove excess probe, and detection of specific labeling of the chromosome target is performed using standard techniques.

For example, a biotinylated probe can be detected using fluorescein-labeled avidin or avidin-alkaline phosphatase. For fluorochrome detection, the fluorochrome can be detected directly, or the samples can be incubated, for example, with fluorescein isothiocyanate (FITC)-conjugated avidin. Amplification of the FITC signal can be effected, if necessary, by incubation with biotin-conjugated goat antiavidin antibodies, washing and a second incubation with FITC-conjugated avidin. For detection by enzyme activity, samples can be incubated, for example, with streptavidin, washed, incubated with biotin-conjugated alkaline phosphatase, washed again and pre-equilibrated (e.g., in alkaline phosphatase (AP) buffer). For a general description of in situ hybridization procedures, see, e.g., U.S. Pat. No. 4,888,278.

Numerous procedures for FISH, CISH, and SISH are known in the art. For example, procedures for performing FISH are described in U.S. Pat. Nos. 5,447,841; 5,472,842; and 5,427,932; and for example, in Pirlkel et al., Proc. Natl. Acad. Sci. 83:2934-2938, 1986; Pinkel et al., Proc. Natl. Acad. Sci. 85:9138-9142, 1988; and Lichter et al., Proc. Natl. Acad. Sci. 85:9664-9668, 1988. CISH is described in, e.g., Tanner et al., Am..1. Pathol. 157:1467-1472, 2000 and U.S. Pat. No. 6,942,970. Additional detection methods are provided in U.S. Pat. No. 6,280,929.

Numerous reagents and detection schemes can be employed in conjunction with FISH, CISH, and SISH procedures to improve sensitivity, resolution, or other desirable properties. As discussed above probes labeled with fluorophores (including fluorescent dyes and QUANTUM DOTS®) can be directly optically detected when performing FISH. Alternatively, the probe can be labeled with a nonfluorescent molecule, such as a hapten (such as the following non-limiting examples: biotin, digoxigenin, DNP, and various oxazoles, pyrrazoles, thiazoles, nitroaryls, benzofurazans, triterpenes, ureas, thioureas, rotenones, coumarin, courmarin-based compounds, Podophyllotoxin, Podophyllotoxin-based compounds, and combinations thereof), ligand or other indirectly detectable moiety. Probes labeled with such non-fluorescent molecules (and the target nucleic acid sequences to which they bind) can then be detected by contacting the sample (e.g., the cell or tissue sample to which the probe is bound) with a labeled detection reagent, such as an antibody (or receptor, or other specific binding partner) specific for the chosen hapten or ligand. The detection reagent can be labeled with a fluorophore (e.g., QUANTUM DOT®) or with another indirectly detectable moiety, or can be contacted with one or more additional specific binding agents (e.g., secondary or specific antibodies), which can be labeled with a fluorophore.

In other examples, the probe, or specific binding agent (such as an antibody, e.g., a primary antibody, receptor or other binding agent) is labeled with an enzyme that is capable of converting a fluorogenic or chromogenic composition into a detectable fluorescent, colored or otherwise detectable signal (e.g., as in deposition of detectable metal particles in SISH). As indicated above, the enzyme can be attached directly or indirectly via a linker to the relevant probe or detection reagent. Examples of suitable reagents (e.g., binding reagents) and chemistries (e.g., linker and attachment chemistries) are described in U.S. Patent Application Publication Nos. 2006/0246524; 2006/0246523, and 2007/01 17153.

It will be appreciated by those of skill in the art that by appropriately selecting labelled probe-specific binding agent pairs, multiplex detection schemes can be produced to facilitate detection of multiple target nucleic acid sequences (e.g., genomic target nucleic acid sequences) in a single assay (e.g., on a single cell or tissue sample or on more than one cell or tissue sample). For example, a first probe that corresponds to a first target sequence can be labelled with a first hapten, such as biotin, while a second probe that corresponds to a second target sequence can be labelled with a second hapten, such as DNP. Following exposure of the sample to the probes, the bound probes can be detected by contacting the sample with a first specific binding agent (in this case avidin labelled with a first fluorophore, for example, a first spectrally distinct QUANTUM DOT®, e.g., that emits at 585 mn) and a second specific binding agent (in this case an anti-DNP antibody, or antibody fragment, labelled with a second fluorophore (for example, a second spectrally distinct QUANTUM DOT®, e.g., that emits at 705 mn). Additional probes/binding agent pairs can be added to the multiplex detection scheme using other spectrally distinct fluorophores. Numerous variations of direct, and indirect (one step, two step or more) can be envisioned, all of which are suitable in the context of the disclosed probes and assays.

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

The nucleic acid primers or probes used in the above amplification and detection method may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In a particular embodiment, the methods of the invention comprise the steps of providing total RNAs extracted from cumulus cells and subjecting the RNAs to amplification and hybridization to specific probes, more particularly by means of a quantitative or semiquantitative RT-PCR.

In another preferred embodiment, the expression level is determined by DNA chip analysis. Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art (see e.g. the review by Hoheisel, Nature Reviews, Genetics, 2006, 7:200-210).

Expression level of a gene may be expressed as absolute expression level or normalized expression level. Typically, expression levels are normalized by correcting the absolute expression level of a gene by comparing its expression to the expression of a gene that is not a relevant for determining the cancer stage of the patient, e.g., a housekeeping gene that is constitutively expressed. Suitable genes for normalization include housekeeping genes such as the actin gene ACTB, ribosomal 18S gene, GUSB, PGK1 and TFRC. This normalization allows the comparison of the expression level in one sample, e.g., a patient sample, to another sample, or between samples from different sources.

In another embodiment, detection of mutations or gene copy-number amplification related to the β8 integrin gene or RNA of patients can be performed. Several techniques can be used like PCR, sequencing methods (Sanger, pyrosequencing, next generation sequencing (NGS) of targeted regions or whole genome). These techniques can be used to find altered β8 integrin in patient glioblastoma. Gene Copy Number can also be used to find amplifications or deletions of one or several exons of the β8 integrin gene or of the total gene leading to protein inactivation or overexpression which may appear in glioblastoma. In this way, techniques like qPCR, MLPA, specific hybridization (FISH, CISH...) and NGS can be used.

The present invention also relates to β8 integrin gene or protein as a biomarker for the diagnosis of resistant glioblastoma.

Another object of the invention relates to a method for treating resistant glioblastoma comprising the steps of i) determining the level expression of the marker β8 integrin in a tumor sample obtained from a patient, ii) comparing the expression levels determined at step i) with predetermined reference values and iii) administering the subject a therapeutically effective amount of a compound which is an antagonist of β8 integrin or an inhibitor of the expression of β8 integrin as described above when the levels determined at step i) is higher than its predetermined reference value.

The invention will be further illustrated by the following figures and examples. However, these examples and figures should not be interpreted in any way as limiting the scope of the present invention.

### FIGURES:

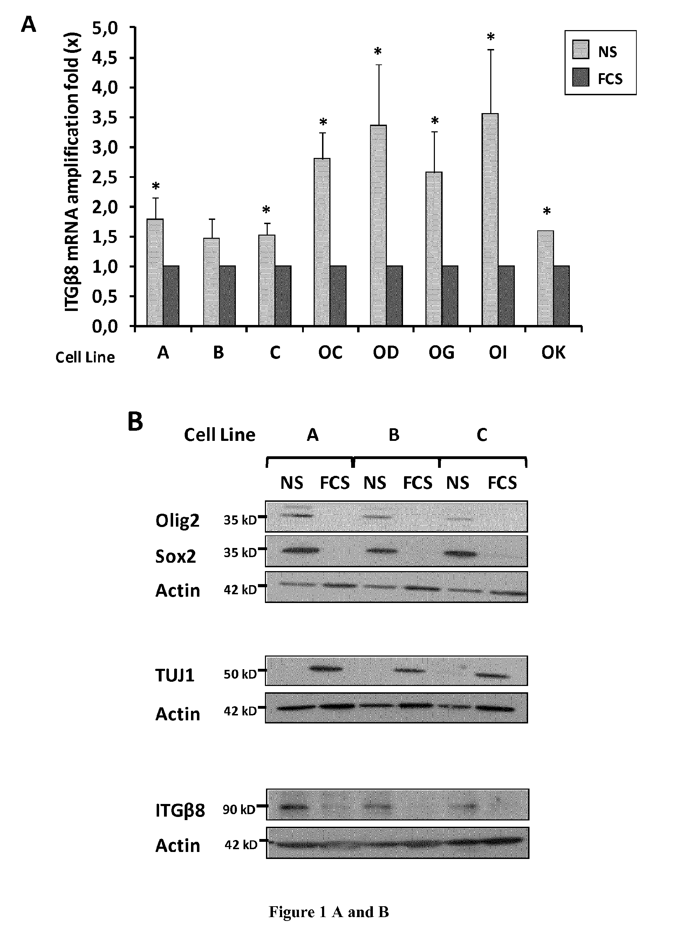

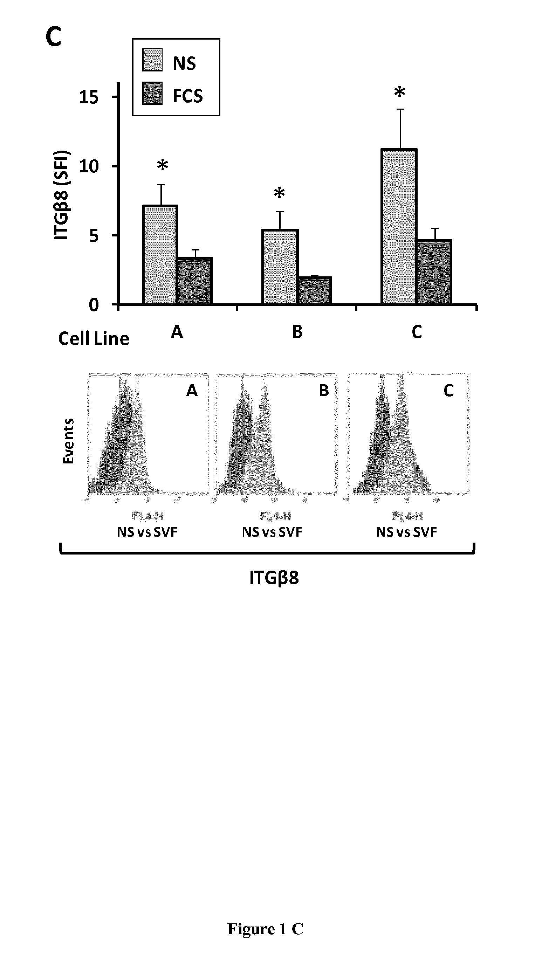

**Figure 1****: β8 integrin (ITGβ8) is overexpressed in GSC compared to GBM differentiated cells.** GSC-enriched neurosphere (NS) cell lines isolated from several patient tumors were maintained in stem cell medium or allowed to differentiate as adherent GBM cells for 15 days in FCS-enriched medium. (A) Real-time Quantitative PCR (qPCR) analysis of the ITGβ8 expression in NS cells and in FCS-differentiated GBM cells. Shown are the fold inductions expressed as means ± SEM of at least four independent experiments. *P < 0.05 compared with the related FCS condition. (B) Western-blot analysis of the ITGβ8 expression in NS cells and in FCS-differentiated cells in comparison with the classic stem (Olig2, Sox2) and differentiation (TUJ1) markers. Equal gel loading and transfer efficiency were checked with anti-actin antibody. Blots were representative of at least three independent experiments in 3 patient cell lines (A, B and C). (C) Immunofluorescence analysis performed by FACS for membrane ITGβ8 expression. The SFI (Specific Fluorescence Index) allowed to evaluate the marker expression level (see Material and Methods). Results are expressed as the means ± SEM of at least five independent experiments. *P < 0.05 compared with the related FCS condition. For each patient cell line (A, B and C), representative FACS plot overlays were depicted.

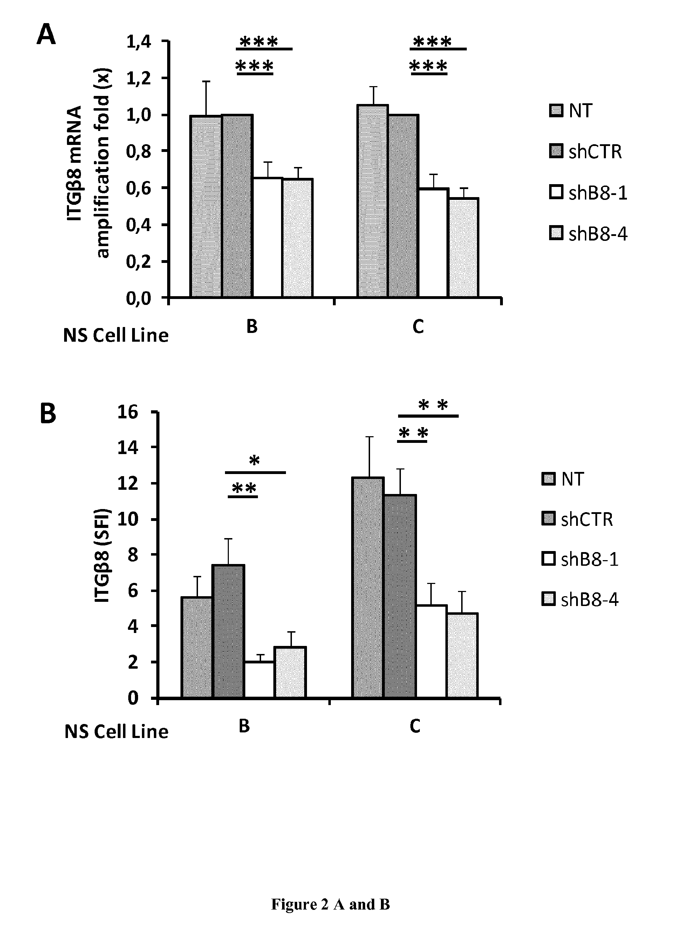

**Figure 2****: ITGβ8 is efficiently downregulated in GSC by specific shRNA targeting.** GSC-enriched NS cells isolated from two patient tumors (B and C) were transfected or not (NT) for 4 days with two different ITGβ8-targeting shRNA (shB8-1 and -4) or with a negative control shRNA (shCTR). ITGβ8 expression levels were then analyzed in NS cells at the mRNA or protein level either by (A) qPCR or (B) FACS immunofluorescence, respectively. Results are expressed as the means ± SEM of at least four independent experiments. * p ≤ 0.05, ** p ≤ 0.01, *** p ≤ 0.001 compared with the related shCTR condition.

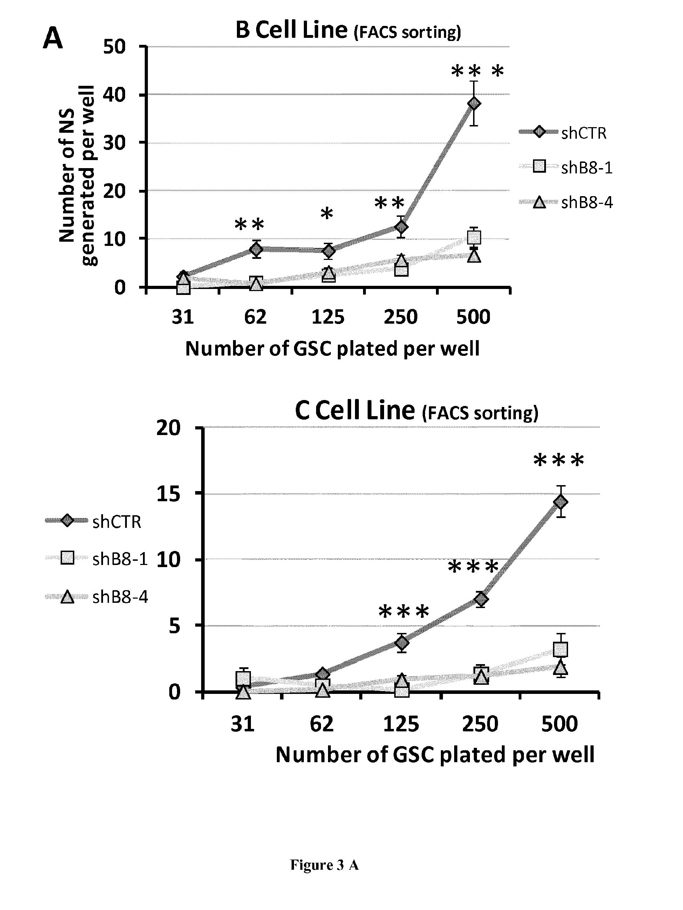

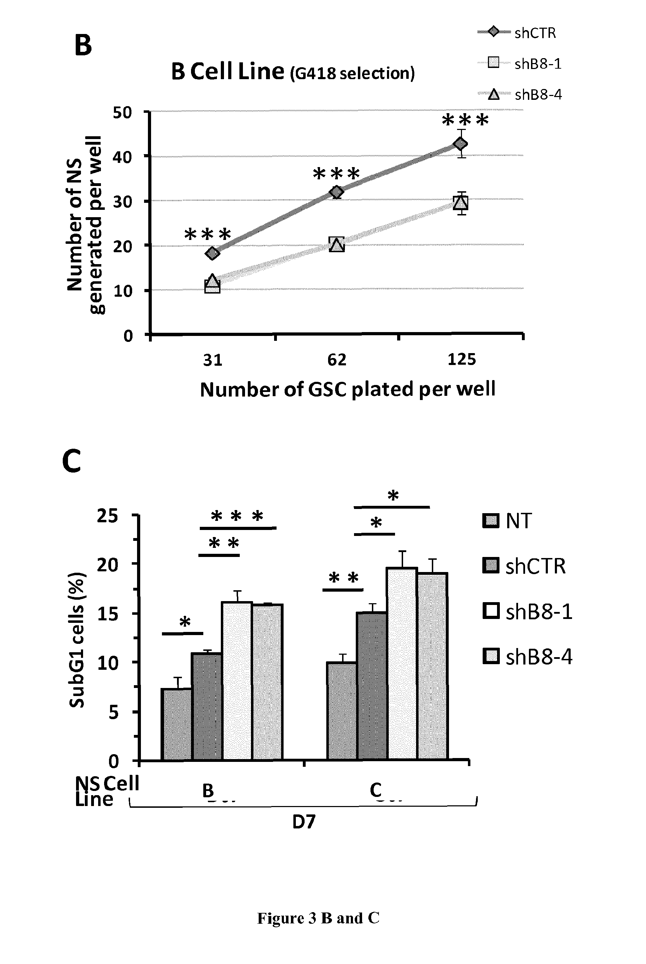

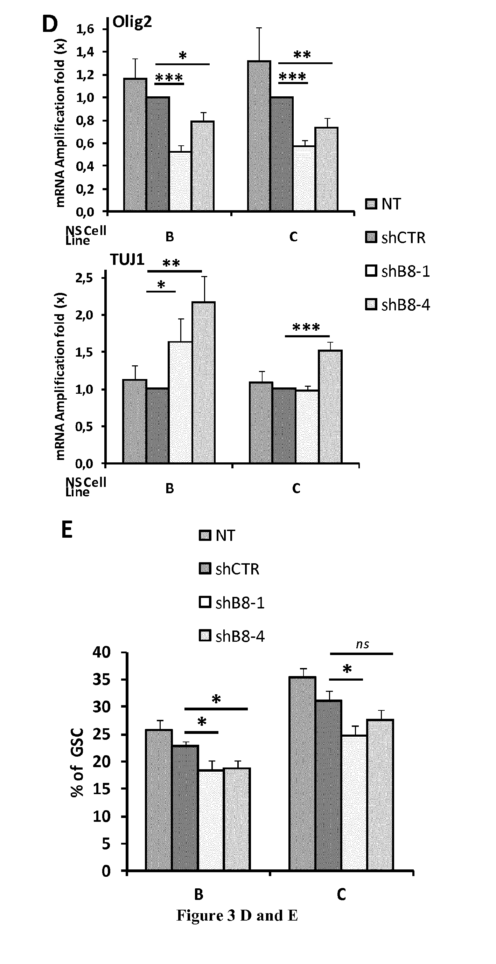

**Figure 3****: shRNA-mediated ITGβ8 knock-down impairs GSC stemness and viabilty.** GSC (B and C cell lines) were transfected or not (NT) with two different ITGβ8-targeting shRNA (shB8-1 and -4) or with a negative control shRNA (shCTR). (A) 4 days after transfection, GSC were subjected to a FACS sorting of either GFP+/ITGB8+ or GFP+/ITGB8- GSC for the shCTR or the shB8 conditions, respectively. These sorted GSC were immediately plated in 96-well plates at different low cell densities to study their ability to generate primary NS through limiting dilution assays. (B) A similar limiting dilution assay was performed in B cell line to assess the secondary NS forming ability of transfected GSC after selection of the shRNA-positive cells by G418 during at least 20 days. Results (A-B) are expressed as the means ± SEM of at least three independent experiments. *P < 0.05. **P < 0.01, ***P < 0.001 compared with the related shCTR condition. (C) 7 days after transfection, GSC were subjected to subG1 analysis by FACS in order to determine the percentage of non-viable cells. (D) mRNA of transfected GSC were extracted 4 days post-transfection to perform qPCR analysis of the expression of two stem (Olig2) and differentiation (TUJ1) markers. (E) These transfected cells were processed by FACS 4 days after transfection to analyze the percentage of the GSC population (see Material and Methods). Results (C-E) are expressed as the means ± SEM of at least four independent experiments. *P < 0.05. **P < 0.01, ***P < 0.001.

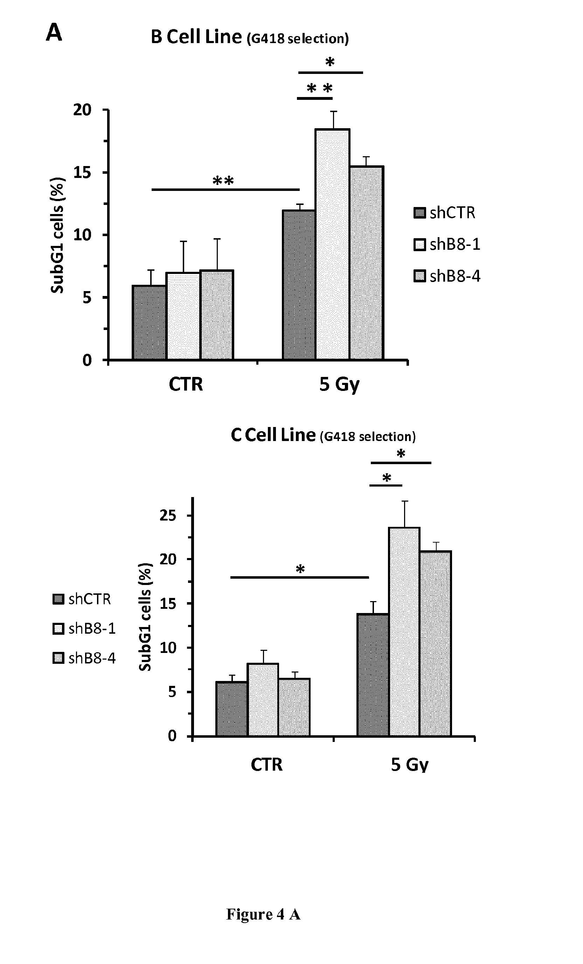

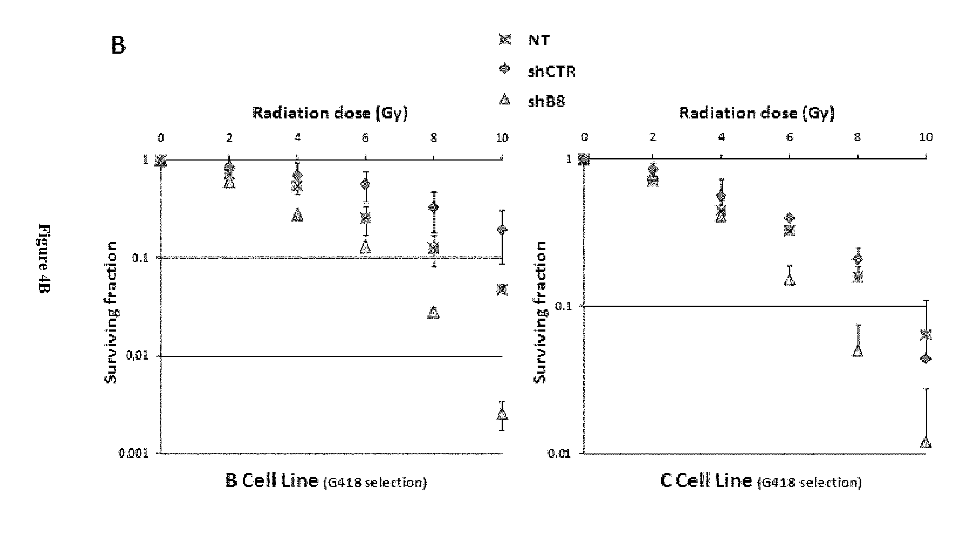

**Figure 4****: shRNA-mediated ITGβ8 knock-down radiosensitizes the GSC population.** GSC (B and C cell lines) were transfected with two different ITGβ8-targeting shRNA (shB8-1 and -4) or with a negative control shRNA (shCTR) and then selected by G418 during at least 20 days. These established stable NS cell lines were then dissociated and irradiated by the indicated dose of ionizing radiations. (A) 4 days after irradiation (5 Gy), cells were subjected to subG1 analysis by FACS in order to determine the percentage of non-viable cells. (B) Prior to irradiation, parental (NT) or stably transfected NS were dissociated and plated in 96-well-plates (500 cells/well) in order to determine the surviving fraction by clonogenic assay. Cells were kept in culture during 10 days and the number of surviving NS clones were determined by microscopy. Results (A-B) are expressed as the means ± SEM of at least three independent experiments. *P < 0.05. **P < 0.01 compared with the related shCTR condition.

### EXAMPLE:

### Material & Methods

### Human tumor collection

The study was conducted on newly diagnosed GBM tumor samples isolated from 8 different patients to establish 8 primary neurosphere (NS) cell lines. These samples were all obtained after written informed consent from patients admitted to the Neurosurgery Department at Toulouse University Hospital and processed in accordance with the Institution's Human research Ethics Committee. Tumors used in this study were histologically diagnosed as grade IV astrocytoma according to the WHO criteria.

### Cell culture

The GBM samples were processed as described by Avril et al in order to obtain the corresponding primary neurosphere (NS) cell lines shown to be enriched in GSC. Neurosphere GSC lines were maintained in DMEM-F12 (Lonza) supplemented with B27 and N2 (Invitrogen), 25 ng/ml of FGF-2 and EGF (Peprotech) at 37°C in 5% CO2 humidified incubators. All GSC lines were used for the experiments in this Stem Cell Medium between the 2nd and 12th passages in order to avoid any stem cell characteristic loss. Forced differentiation was performed according to previous published protocol adapted as follows. Briefly, the dissociated NS cells were cultured and plated as adherent monolayer (7.5x10³ cells/cm²) in DMEM-F12 only supplemented with 10% FCS (FCS medium) for at least 15 days to insure an optimum differentiation.

### Quantitative real-time RT-PCR

Total RNA was isolated either from primary NS, FCS-differentiated cells or from NS cells after transfection using RNeasy kit (Qiagen) and then reverse-transcribed using iScript cDNA synthesis kit (Bio-Rad). Real-time qPCR reactions were carried out using Evagreen dye and ABI-Stepone+ Detection System (Applied Biosystems). GAPDH was used as endogenous control in the ΔCt analysis. Amplification folds were measured by the 2^{-ΔΔCt} method. Different primers (Eurogentec) were used in this study.

### Western blotting

Cells were lysed in RIPA buffer complemented with cocktails of protease and phosphatase inhibitors (Sigma). 25µg of proteins were then separated on a 10% or 12.5% SDS-PAGE, electroblotted onto PVDF membranes (Amersham), which were blocked with 10% milk. The primary antibodies used for this study are listed in Table 1.

**Table 1: Immunoblot antibodies.**

| **PROTEIN** | **Primary Antibody** |
|---|---|
| Actin (*Human*) | Mouse anti-Actin (MAB1501, 1/10000, Millipore) |
| ITGβ8 (*Human*) | Rabbit anti-ITGβ8 (ab80673), 1/1000, Abcam) |
| Olig2 (*Human*) | Rabbit anti-Olig2 (AB9610, 1/2500, Millipore) |
| Sox2 (*Human*) | Rabbit anti-Sox2 (ab92494, Abcam) |
| UJ1 (*Human*) | Mouse anti-TUJ1 (MAB1637, 1/1000, Millipore) |

### Flow cytometry immunofluorescence assay

Direct immunofluorescence assay was performed by FACS as previously described. For all samples, 2x10⁵ cells were incubated for 30 min in PBS with 10% BSA at 4°C to avoid nonspecific binding, and then incubated with appropriate APC-conjugated anti-ITGβ8 antibody (R&D, 1/20) for 40 min at 4°C. Fluorescence related to immunolabeling was measured using a FACSCalibur flow cytometer (BD Biosciences). Each measurement was conducted on at least 7,000 events, acquired on CellQuest software (BD Biosciences) and analyzed with VenturiOne software (AppliedCytometry). To evaluate the marker expression, we determined the specific fluorescence index (SFI) using the mean fluorescence intensity (MFI). The SFI was calculated as previously described with the following formula SFI = (MFI antibody - MFI isotype control) / MFI isotype control.

### SubG1 population quantification

Dead cells were quantified using FACS by determining the percentage of cells with subG1-DNA content. This subG1 population was analyzed after cell permeabilization and subsequent Propidium Iodide staining, as previously described. SubG1 measurements were conducted on at least 10,000 events, acquired on CellQuest software (BD Biosciences) and analyzed with VenturiOne software (Applied Cytometry).

### shRNA transfection

Neurospheres from 2 different patient cell lines (B and C) were dissociated and 7x10⁵ cells per well were plated in 6 well-plates before being transfected using Fugene HD (3/1 ratio, Promega). 2 different shRNA sequences targeting ITGβ8 (shβ8-1 and -4, Qiagen) were used to specifically knock-down ITGβ8 expression in GSC (Table 2). An off-target shRNA (shCTR, Qiagen) was used as a negative control. These shRNA were either coupled to a GFP-reporter gene or to a Neomycin-resistance gene for short-term transfection or stable selection in G418 (1 mg/mL, 20 days, Merck), respectively. Medium replacement was done 24h after transfection in order to avoid any unspecific toxicity.

**Table 2: shRNA sequences.**

| **Name** | **Target** | **Reporter or Resistance** | **Sequence** | **SEQ ID** |
|---|---|---|---|---|
| shCTR | Off-target | GFP or Neomvcin | GGAATCTCATTCGATGCATAC | SEQ ID N°: 1 |
| shβ8-1 | ITGβ8 | GFP or Neomvcin | CCAAGCTACTTGAGAATATTT | SEQ ID N°: 2 |
| shβ8-4 | ITGβ8 | GFP or Neomycin | TCTCGCTCTTGATAGCAAATT | SEQ ID N°: 3 |

### GSC gating and sorting by FACS

In order to discriminate by FACS the GSC population from the more differentiated population, both present among the NS cells, we adapted a previously published protocol. Briefly, NS cells were dissociated and 2x10⁵ cells were analyzed by flow cytometry. The percentages of each population were subsequently quantified according to their specific FSC/SSC patterns. Prior to this, a pre-gating was performed to avoid cell debris in the analysis.

For the measurement of the NS formation capacity by limiting dilution assay, cells were transfected for 4 days with GFP-coupled shRNA (shB8-1/-4 or shCTR) before being sorted by FACS (Beckman MoFlo Astrios). This sorting was performed on the GSC population present within the NS cells and was designed to only sort the GFP-positive cells with normal (for shCTR condition) or negative (for shB8-1/-4 conditions) expression for ITGβ8. Cells were then collected and plated for limiting dilution assay.

### Neurosphere formation assays

Cells obtained after GFP-coupled shRNA transfection were sorted by FACS as described above and immediately plated in 96-well plates at different low cellular densities (1 to 500 cells/well) in order to assess their ability to generate primary NS through limiting dilution assays. After 15 days, the generated NS were counted by microscopy in each well.

When cells were transfected by shRNA vectors carrying the Neomycin-resistance gene, cells were treated 4 days after transfection by G418 during at least 20 days in order to select the positive clones, capable of forming NS during the selection process. These NS cells were then dissociated and plated in 96-well plates to perform similar limiting dilution assays and measure their ability to generate secondary NS.

### Irradiation

The stable NS clones selected in G418 (shB8-1/-4 or shCTR) were subjected or not to different doses of irradiation (Gamma-cell Exactor 40, Nordion) in order to evaluate the radiosensitizing effect of ITGβ8 knock-down in GSC through subG1 analysis and clonogenic assays.

### Clonogenic assays

Prior to irradiation, parental or stably transfected NS (shB8 or shCTR clones) were dissociated and plated in 96-well-plates (500 cells/well) in order to determine the surviving fraction by clonogenic assay. Cells were then irradiated or not with increasing doses of ionizing radiations (0 to 10 Gy) and kept in culture during 10 days. Finally, the number of surviving NS clones was determined by microscopy.

### Statistical analysis

The results are presented as means ± SEM of at least three independent experiments. Significant differences (*P < 0.05, **P < 0.01 and ***P < 0.001) were evaluated with the Student t-test. Log. Western-blots and FACS plots are representative of at least three different experiments.

### Results

### 1. ITGβ8 overexpression in GSC compared to GBM differentiated cells

We screened by RT-qPCR the expression level of ITGβ8 mRNA in 8 different patient GSC cell lines, cultured as neurospheres (NS) in stem medium, compared to their differentiated counterparts, kept in FCS-enriched differentiation medium (Figure 1A). We observed that this integrin was significantly overexpressed in all GSC cell lines (1.5 to 3.5 fold according to the patient). Moreover, we confirmed on 3 patient cell lines that the protein level of ITGβ8 was also overexpressed in GSC-enriched NS in comparison with their differentiated counterparts. This overexpression was demonstrated in whole cell lysates, as shown by western-blot (Figure 1B), and at the membrane cell surface, as observed by flow cytometry (Figure 1C). In order to fully establish the preferential expression of this particular integrin in GSC, we additionally performed western-blot analysis of several stem (Olig2, Sox2) and differentiation (TUJ1) markers and confirmed that NS cells specifically expressed these stem markers and failed to express the neuronal marker TUJ1 (Figure 1B). We can conclude that ITGβ8 is selectively express at the membrane of glioblastoma stem-like cells and barely present in differentiated GBM cells.

### 2. ITGβ8 inhibition by shRNA greatly alter GSC viability and stemness

We subsequently designed a shRNA-based strategy to selectively downregulate ITGβ8 in GSC in order to assess its functions in this subpopulation of undifferentiated cells. We used two different ITGβ8-targeting shRNA sequences (shB8-1 and -4) or a negative control shRNA (shCTR) and transfected NS-dissociated cells for 4 days. We observed in two different patient NS cell lines (B and C) that shB8-1 and -4 sequences potently inhibited the expression of ITGβ8 mRNA level (Figure 2A) and the presence of this integrin at the cell membrane surface (Figure 2B) in comparison with both the non-transfected (NT) or shCTR conditions. One of the main characteristic of cancer stem cells is their self-renewal ability in limiting dilution condition. We assessed this sternness-associated parameter by measuring the NS forming capacity of GSC at low cell density after ITGβ8-targeting. We demonstrated that GFP-coupled shB8-1 and -4 dramatically abrogated the NS forming capacity of ITGβ8-/GFP+ GSC compared to the ITGβ8+/GFP+ shCTR GSC in FACS-sorted cells from the B and C patient NS cell lines (Figure 3A). Moreover, transfection of the same shB8-1 and -4 sequences coupled to the neomycin-resistance gene and subsequent selection by G418 induced a similar drop in the NS generation ability at low density compared to the shCTR condition (Figure 3B).
This major alteration of the GSC *in vitro* self-renewal ability in response to ITGβ8 inhibition can be mediated by several causes such as viability loss. We notably observed that ITGβ8-targeting by shB8-1 and -4 induced 7 days after transfection a significant increase of the subG1 cell population in NS compared to NT and shCTR conditions, which may be associated with the induction of a pro-apoptotic mechanism in transfected ITGβ8-negative GSC (Figure 3C). In addition, a decrease of the GSC self-renewal ability could also be linked to a weakening of their stem phenotype due to ITGβ8 knock-down. In that way, we demonstrated that shRNAs against ITGβ8 induce 4 days after transfection a significant decrease of the stem factor Olig2 and an increase of the TUJ1 differentiation marker at the mRNA level (Figure 3D). These variations in stem and differentiation marker expression were associated to a noticeable decrease of the GSC subpopulation within the NS structures in response to ITGβ8, as defined by flow cytometry analysis in B and C patient cell lines (Figure 3E). Altogether, these observations suggest that ITGβ8 knock-down may induce a differentiation process in GSC associated to an alteration of their stemness characteristics, such as self-renewal ability and stem markers, and to the induction of cell death.

### 3. ITGβ8 inhibition by shRNA radiosensitizes the GSC population

GSC are, among the GBM cellular heterogeneity, one of the most radioresistant tumoral subpopulation. They were shown to resist to the deleterious effects of ionizing radiations (IR) by several mechanisms such as a better efficiency of DNA damage repair systems or a higher level of anti-apoptotic or pro-survival factors. However, the selective expression of particular integrins in GSC and their roles on GSC radioresistance, which are largely unknown in the literature, appeared to us of potential interest as part of a combined therapeutic approach. As a consequence, we then studied the combined effect of ITGβ8 inhibition by shRNA on GSC radioresistance. For that purpose, we submitted G418-selected shB8-1 and -4 clones (B and C cell lines) to a toxic IR dose of 5 Gy for 4 days and measured that ITGβ8-targeting significantly increased the level of subG1-positive cells compared to the shCTR condition in response to IR (Figure 4A). Moreover, to fully demonstrate the radiosensitizing effect of ITGβ8 downregulation in GSC, we performed clonogenic assays at low density in 96 well plates and measured the NS generation potential of shCTR or shB8 stably-transfected GSC (B and C patients) in response to increasing doses of IR (up to 10 Gy). As depicted in Figure 4B, we observed that the shRNA-mediated invalidation of ITGβ8 induced a significant decrease in the NS surviving fraction in response to IR (6 to 10 Gy) in comparison with shCTR cells or non-transfected NT cells.

Altogether, these results demonstrate that (i) ITGβ8 is specifically expressed by GSC compared to more differentiated GBM cells, (ii) this integrin could mediate in GSC a pro-survival signal associated to the maintenance of a stem phenotype and (iii) the specific inhibition of this GSC-specific target could radiosensitize GBM-stem cells, known as a really radioresistant subpopulation in GBM able to favor the recurrence of these aggressive and invasive brain tumors.

### REFERENCES:

Throughout this application, various references describe the state of the art to which this invention pertains. The disclosures of these references are hereby incorporated by reference into the present disclosure.
Cambier S, Gline S, Mu D, Collins R, Araya J, Dolganov G, Einheber S, Boudreau N, Nishimura SL. Integrin alpha(v)beta8-mediated activation of transforming growth factor-beta by perivascular astrocytes: an angiogenic control switch. Am J Pathol. 2005 Jun;166(6):1883-94.
Cheng L, Bao S, Rich JN. Potential therapeutic implications of cancer stem cells in glioblastoma. Biochem Pharmacol. 2010 Sep 1;80(5):654-65. doi: 10.1016/j.bcp.2010.04.035. Epub 2010 May 10.
Minagawa S et al. Selective targeting of TGF-β activation to treat fibroinflammatory airway disease. Sci Transl Med. 2014 Jun 18;6(241):241ra79. doi: 10.1126/scitranslmed.3008074.
Reyes SB1, Narayanan AS, Lee HS, Tchaicha JH, Aldape KD, Lang FF, Tolias KF, McCarty JH. αvβ8 integrin interacts with RhoGDI1 to regulate Rac1 and Cdc42 activation and drive glioblastoma cell invasion. Mol Biol Cell. 2013 Feb;24(4):474-82. doi: 10.1091/mbc.E12-07-0521. Epub 2013 Jan 2.
Sheldrake HM1, Patterson LH. Strategies to inhibit tumor associated integrin receptors: rationale for dual and multi-antagonists. J Med Chem. 2014 Aug 14;57(15):6301-15.
Stupp R et al. Cilengitide combined with standard treatment for patients with newly diagnosed glioblastoma with methylated MGMT promoter (CENTRIC EORTC 26071-22072 study): a multicentre, randomised, open-label, phase 3 trial. Lancet Oncol. 2014 Sep;15(10):l 100-8. doi: 10.1016/S1470-2045(14)70379-1. Epub 2014 Aug 19.
Thomas AA1, Omuro A. Current Role of Anti-Angiogenic Strategies for Glioblastoma. Curr Treat Options Oncol. 2014 Aug 31.

## Claims

1. A compound which is an antagonist of β8 integrin or an inhibitor of the expression of β8 integrin for use in the treatment of resistant glioblastoma.

2. A i) compound according to claim1 and ii) a radiotherapy, and iii) TMZ as a combined preparation for simultaneous, separate or sequential use in the treatment of resistant glioblastoma.

3. A i) compound according to claim 1 and ii) a chemotherapeutic agent, as a combined preparation for simultaneous, separate or sequential for use in the treatment of resistant glioblastoma.

4. A i) compound according to claim 1 and ii) a radiotherapy, and iii) a chemotherapeutic agent, as a combined preparation for simultaneous, separate or sequential for use in the treatment of resistant glioblastoma.

5. A i) compound according to claim 1 and ii) a radiotherapy, as a combined preparation for simultaneous, separate or sequential for use in the treatment of resistant glioblastoma.

6. A compound according to claim 1 or a combined preparation according to claims 2, 3, 4 or 5 wherein the compound is an antagonist of β8 integrin.

7. A compound according to claim 6 or a combined preparation according to claim 6 wherein the antagonist is an antibody anti- β8 integrin.

8. A pharmaceutical composition comprising an effective dose of an antagonist of β8 integrin or an inhibitor of the β8 integrin expression, for use in the treatment of resistant glioblastoma.

9. An ex vivo method for diagnosing resistant glioblastoma, comprising the step of determining the level expression of the marker β8 integrin in a tumor sample obtained from a patient.


SEQUENCE LISTING

<110>  INSERM

<120>  NEW METHOD FOR TREATING RESISTANT GLIOBLASTOMAS

<130>  BIO14256 LEMARIE

<160>  3

<170>  PatentIn version 3.3

<210>  1
<211>  21
<212>  DNA
<213>  Artificial

<220>
<223>  shCTR

<400>  1
ggaatctcat tcgatgcata c                                                21


<210>  2
<211>  21
<212>  DNA
<213>  Artificial

<220>
<223>  shß8-1

<400>  2
ccaagctact tgagaatatt t                                                21


<210>  3
<211>  21
<212>  DNA
<213>  Artificial

<220>
<223>  shß8-4

<400>  3
tctcgctctt gatagcaaat t                                                21